(19) Europäisches Patentamt
European Patent Office
Office européen des brevets

(11) Publication number: **0 197 798 B1**

# (12) EUROPEAN PATENT SPECIFICATION

(45) Date of publication of patent specification: **27.12.90**

(51) Int. Cl.[5]: **C 07 K 7/06,** C 07 K 7/20, A 61 K 37/02

(21) Application number: **86302621.7**

(22) Date of filing: **09.04.86**

## (54) Peptides.

(30) Priority: **09.04.85 US 721330**

(43) Date of publication of application:
**15.10.86 Bulletin 86/42**

(45) Publication of the grant of the patent:
**27.12.90 Bulletin 90/52**

(84) Designated Contracting States:
**AT BE CH DE FR GB IT LI LU NL SE**

(56) References cited:
**EP-A- 171 477**
**EP-A-0 038 135**
**EP-A-0 049 628**
**EP-A-0 081 877**
**EP-A-0 097 031**
**EP-A-0 100 218**
**EP-A-0 225 746**
**GB-A-2 009 182**

**CHEMICAL ABSTRACTS, vol. 90, no. 25, 18th June 1979, page 80, abstract no. 197991m, Columbus, Ohio, US; K. CHANNABASAVAIAH et al.: "New analogs of luliberin which inhibit ovulation in the rat", & BIOCHEM. BIOPHYS. RES. COMMUN. 1979, 86(4), 1266-73**

(73) Proprietor: **ADMINISTRATORS OF THE TULANE EDUCATIONAL FUND**
**1430 Tulane Avenue Tulane University**
**New Orleans Louisiana 70112 (US)**

(72) Inventor: **Coy, David H.**
**4319 Perrier Street**
**New Orleans Louisiana 70115 (US)**
Inventor: **Moreau, Jacques-Pierre**
**159 Westboro Road**
**Upton Massachusetts 01568 (US)**

(74) Representative: **Deans, Michael John Percy et al**
**Lloyd Wise, Tregear & CO. Norman House**
**105-109 Strand**
**London WC2R OAE (GB)**

(56) References cited:
**JOURNAL OF MEDICINAL CHEMISTRY, vol. 27, 1984, pages 1170-1174, American Chemical Society; J.J. NESTOR et al.: "Luteinizing hormone-releasing hormone antagonists containing very hydrophobic amino acids"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 197 798 B1

**Description**

This invention relates to peptides.

A number of luteinizing hormone releasing hormone (LH-RH) analogs have been described which inhibit the release of LH-RH, a peptide hormone having the formula pGlu-His-Trp-Ser-Tyr-Gly-Leu-Arg-Pro-Gly-$NH_2$.

For example, Coy et al. U.S. Pat. No. 4,431,635, the disclosure of which is hereby incorporated by reference, described LH-RH analogs having the general formula X-$R^1$-$R^2$-$R^3$-Ser-Tyr-$R^4$-Leu-Arg-Pro-$R^5$-$NH_2$, in which X can be Ac; $R^1$ and $R^4$, independently, can be D-Trp or D-p-X-Phe, where X is a halogen or methyl group; $R^2$ can be D-p-X-Phe; $R^3$ can be D-Trp; and $R^5$ can be Gly or D-Ala.

Channabasavaiah et al. (1979) B.B.R.C. *86*:1266 report the effect of D-amino acids at position 1 in conjunction with D-amino acids at positions 2, 3 and 6.

Nestor et al. (1984) J. Med. Chem. *27*:1170 report that the presence of the hydrophobic residues, specifically halogenated Phe at position 2 and 3-(2-naphthyl)-D-Ala, D-Trp, or D-Phe at position 3, result in an increase in activity.

EP—A—0100218 (The Salk Institute for Biological Studies) teaches analogs with a substituted Phe residue at position 2 and D-Trp at position 3.

EP—A—0038135 (The Salk Institute for Biological Studies) discloses analogs with the hydrophobic residue D-Phe at positions 2 and 3.

EP—A—0097031 (Syntex (USA) Inc.) reports that the activity of analogs with replacements at position 2 could be augmented by replacement of the Gly residue at position 6 with a non-natural guanido-substituted, amidine, or tertiary or quaternary amine water soluble amino acid residue.

EP—A—0049628 (Syntex (USA) Inc.) reports the replacement of glycine at position 6 with a series of non-natural D-lipophilic amino acids.

The present invention arises from our work seeking to provide improved LH-RH analogs and provides decapeptides of the formula: N-Ac-$A^1$-$A^2$-$A^3$-Ser-Tyr-$A^4$-$A^5$-$A^6$-$A^7$-$A^8$, wherein $A^1$ is D-β-Nal, D-p-X-Phe (where X is Cl, F, Br, or $CH_3$), D-Trp, or D-Phe; $A^2$ is D-p-X-Phe (where X is Cl, F, Br, or $CH_3$), D-Phe, or D-Tyr; $A^3$ is D-Trp, D-Phe, or D-Tyr; $A^4$ is D-Arg, D-β-Naphthyl-Ala, D-Trp, D-Lys, D-homo-Arg, D-diethyl-homo-Arg, or D-p-X-Phe (where X is Cl, F, Br, or $CH_3$), $A^5$ is Phe or Tyr, $A^6$ is Arg or Lys; $A^7$ is Pro or OH-Pro; and $A^8$ is D-Ala, D-Ala-$NH_2$, Gly or Gly-$HN_2$; provided that at least one of $A^2$ or $A^3$ must be D-Phe; or a pharmaceutically acceptable salt thereof.

In one preferred decapeptide, $A^1$ is D-β-Nal, $A^2$ is D-p-Cl-Phe, $A^3$ is D-Phe, $A^4$ is D-Arg, $A^5$ is Phe, $A^6$ is Arg, $A^7$ is Pro, and $A^8$ is D-Ala.

In another preferred decapeptide, $A^1$ is D-β-Nal, $A^2$ is D-Phe, $A^3$ is D-Phe, $A^4$ is D-Arg, $A^5$ is Phe, $A^6$ is Arg, $A^7$ is Pro, and $A^8$ is D-Ala.

In other preferred embodiments, a therapeutically effective amount of the therapeutic decapeptide and a pharmaceutically acceptable carrier substance, e.g., magnesium carbonate or lactose, together form a therapeutic composition for inhibiting the release of sex hormones, particularly LH, induced by LH-RH. This composition can be in the form of a pill, tablet, capsule, liquid, or sustained release tablet for oral administration; a liquid spray for nasal administration; or a liquid for intravenous, subcutaneous, parenteral, or intraperitoneal administration.

Another preferred form for administration is an injectible suspension of the peptide with a bioerodible, biocompatible polymer matrix capable of effecting sustained release of the peptide. Other suitable forms are peptide/polymer implants and transdermal patches.

We have found examples of the decapeptides to be active in inhibiting the LH-RH induced release of LH, and to exhibit a long duration of activity, thus minimizing the amount and frequency of dosages. Furthermore, manufacture is relatively simple and inexpensive. In addition, the peptides have the advantage of being able to be administered orally, a property owing at least in part to their high lipophilicity.

D-Phe at position $A^3$ has been found to be a modification of particular importance in terms of activity, while D-Phe at position $A^2$ provides further cost reduction, compared to D-p-X-Phe at $A^2$, without significant comparative loss of activity. D-Phe at $A^2$ also lessens the irritant effect of the decapeptide. The peptides of the invention have D-Phe at at least one of positions $A^2$ or $A^3$.

Other features and advantages of the invention will be apparent from the following description of the preferred embodiments thereof.

We now describe the structure, synthesis, and use of preferred embodiments of the invention.

The decapeptides of the invention have the general formula N-Ac-$A^1$-$A^2$-$A^3$-Ser-Tyr-$A^4$-$A^5$-$A^6$-$A^7$-$A^8$.

They all have an acetyl group at the amino terminal end in addition to Ser at position 4 and Tyr at position 5. Substitution of non-natural substituents at positions other than $A^2$, $A^3$, $A^4$ and $A^5$ can be used to modify the properties of the compound, and will not prevent the $A^2$ and/or $A^3$ substituents from providing their beneficial effects.

The decapeptides can be provided in the form of pharmaceutically acceptable salts. Examples of preferred salts are those with therapeutically acceptable organic acids, e.g.,. acetic, lactic, maleic, citric, malic, ascorbic, succinic, benzoic, salicylic, methanesulphonic, toluenesulphonic, trifluoroacetic, or pamoic acid, as well as polymeric acids such as tannic acid or carboxymethyl cellulose, and salts with inorganic

acids such as the hydrohalic acids, e.g., hydrochloric acid, sulphuric acid, or phosphoric acid.

The synthesis of N-Ac-D-β-Nal-D-Phe-D-Phe-Ser-Tyr-D-Arg-Phe-Arg-Pro-D-Ala follows.

Other decapeptides of the invention can be prepared by making appropriate modifications of the following synthetic method.

The first step is the preparation of N-acetyl-D-β-Nal-D-Phe-D-Phe-Ser-Tyr-D-tosyl-Arg-Phe-tosyl-Arg-Pro-D-Ala-benzyhydrylamine-resin, as follows.

Benzyhydrylamine-polystyrene resin (Bachem, Inc.) (1.00 g, 0.3 mmole) in the chloride ion form is placed in the reaction vessel of a Beckman 990B peptide synthesizer programmed to perform the following reaction cycle: (a) $CH_2Cl_2$; (b) 33% trifluoroacetic acid in $CH_2Cl_2$ (2 times for 1 and 25 min each); (c) $CH_2Cl_2$; (d) ethanol; (e) $CH_2Cl_2$; (f) 10% triethylamine in $CHCl_3$; and (g) $CH_2Cl_2$.

The neutralized resin is stirred with alpha-t-butoxycarbonyl (Boc)-D-Ala and diisopropylcarbodiimide (1.5 mmol) in $CH_2Cl_2$ for 1 hour and the resulting amino acid resin is then cycled through steps (a) to (g) in the above wash programme. The following amino acids (1.5 mmole) are then coupled successively by the same procedure: Boc-Pro, Boc-Tosyl-Arg, Boc-Phe, Boc-Tosyl-D-Arg, Boc-Tyr, Boc-benzyl-Ser, Boc-D-Phe, Boc-D-Phe, and Boc-D-β-Nal.

After removal of the N-terminal Boc group, the peptide-benzyhydrylamine resin is neutralized and acetylated by treatment with 5% acstic acid in $CH_2Cl_2$. The completed resin is then washed with $CH_3OH$ and air dried.

From the above resin is prepared N-Ac-D-β-Nal-D-Phe-D-Phe-Ser-Tyr-D-Arg-Phe-Arg-Pro-D-Ala, as follows.

A mixture of the above decapeptide resin (1.85 g, 0.5 mmole) and a solution of 4 ml anisole, 100 mg dithiothreitol, and 36 ml hydrogen fluoride is stirred at 0°C for 45 minutes. Excess hydrogen fluoride is evaporated rapidly under a stream of dry nitrogen, after which the free peptide is precipitated and washed with ether.

The peptide is then dissolved in a minimum volume of 50% acetic acid and eluted on a column (2.5×100 mm) of Sephadex G-25 (Sephadex is a Trade Mark). Fractions containing a major component, as determined by u.v. absorption and thin layer chromatography (tlc), are pooled and evaporated to a small volume *in vacuo*. This solution is applied to a column (2.5×50 cm) of octadecylsilane-silica (Whatman LRP-1, 15—20 μm mesh size) which is eluted with a linear gradient of 15—50% acetonitrile in 20% acetic acid in water. Fractions are examined by tlc and analytical high performance liquid chromatography (hplc) and pooled to give maximum purity. Repeated lyophilization of the solution from water gives 117 mg of the product as a white, fluffy powder.

This material is found to be homogeneous by hplc and tlc. Amino acid analysis of an acid hydrolysate confirms the composition of the decapeptide.

N-Ac-D-β-Nal-D-p-Cl-Phe-D-Phe-Ser-Tyr-D-Arg-Phe-Arg-Pro-D-Ala was prepared according to the synthesis described above, substituting Boc-D-p-Cl-Phe for Boc-D-Phe at position $A^2$.

When administered to a mammal (e.g., orally, intravenously, parenterally, nasally, or by suppository), the decapeptides are effective in inhibiting the release of LH induced by LH-RH.

## Claims

1. A decapeptide of the formula: N-Ac-$A^1$-$A^2$-$A^3$-Ser-Tyr-$A^4$-$A^5$-$A^6$-$A^7$-$A^8$, wherein $A^1$ is D-β-Nal, D-p-X-Phe (where X is Cl, F, Br, or $CH_3$), D-Trp, or D-Phe; $A^2$ is D-p-X-Phe (where X is Cl, F, Br, or $CH_3$), D-Phe, or D-Tyr; $A^3$ is D-Trp, D-Phe, or D-Tyr; $A^4$ is D-Arg, D-β-Naphthyl-Ala, D-Trp, D-Lys, D-homo-Arg, D-diethyl-homo-Arg, or D-p-X-Phe (where X is Cl, F, Br, or $CH_3$); $A^5$ is Phe or Tyr; $A^6$ is Arg or Lys; $A^7$ is Pro or OH-Pro; and $A^8$ is D-Ala-, D-Ala-$NH_2$, Gly, or Gly-$NH_2$; provided that at least one of $A^2$ or $A^3$ must be D-Phe; or a pharmaceutically acceptable salt thereof.

2. The decapeptide of Claim 1, wherein $A^1$ is D-β-Nal, $A^2$ is D-p-Cl-Phe, $A^3$ is D-Phe, $A^4$ is D-Arg, $A^5$ is Phe, $A^6$ is Arg, $A^7$ is Pro, and $A^8$ is D-Ala; or a pharmaceutically acceptable salt thereof.

3. The decapeptide of Claim 1, wherein $A^1$ is D-β-Nal, $A^2$ is D-Phe, $A^3$ is D-Phe, $A^4$ is D-Arg, $A^5$ is Phe, $A^6$ is Arg, $A^7$ is Pro, and $A^8$ is D-Ala; or a pharmaceutically acceptable salt thereof.

4. A therapeutic composition comprising a therapeutically effective amount of a decapeptide as defined in any of Claims 1 to 3 or a pharmaceutically acceptable salt thereof, together with a pharmaceutically acceptable carrier substance.

5. The composition of Claim 4, wherein said composition is provided in the form of a pill, tablet, capsule, liquid, or sustained release tablet for oral administration; in the form of a liquid spray for nasal administration; in the form of a liquid capable of being administered intravenously, subcutaneously, parenterally, or intraperitoneally; in the form of a bioerodible, biocompatible implant; or in the form of a transdermal patch.

6. The composition of Claim 4, wherein said composition is provided in the form of an injectible suspension comprising said decapeptide or pharmaceutically acceptable salt thereof, and a bioerodible, biocompatible polymer matrix capable of effecting sustained release of said decapeptide or salt.

7. A decapeptide as claimed in any of Claims 1 to 3, or its pharmaceutically acceptable salt or a composition according to any of Claims 4, 5 or 6, for use in the treatment of precocious puberty, of hormone dependent tumours, for example, malignant and benign prostatic, mammary, ovarian and

testicular tumours, of hirsutism, of acne, of amenorrhea, of endometriosis, or of ovarian and mammary cystic diseases, for use in preventing the growth of mammary tumours, for use in inhibiting the LH-RH induced release of sex hormones, for use in regulating human menopausal gonadotropin luteinizing hormone and follicle-stimulating hormone during perimenopausal and post menopausal periods in women, or for use in female contraceptives.

8. A method of synthesizing a peptide of the formula: N-Ac-$A^1$-$A^2$-$A^3$-Ser-Tyr-$A^4$-$A^5$-$A^6$-$A^7$-$A^8$, wherein $A^1$ is D-β-Nal, D-p-X-Phe (where X is Cl, F, Br, or $CH_3$), D-Trp, or D-Phe; $A^2$ is D-p-X-Phe (where X is Cl, F, Br, or $CH_3$), D-Phe, or D-Tyr; $A^3$ is D-Trp, D-Phe, or D-Tyr; $A^4$ is D-Arg, D-β-Naphthyl-Ala, D-Trp, D-Lys, D-homo-Arg, D-diethyl-homo-Arg, or D-p-X-Phe, (where X is Cl, F, Br, or $CH_3$); $A^5$ is Phe or Tyr; $A^6$ is Arg or Lys; $A^7$ is Pro or OH-Pro; and $A^8$ is D-Ala, D-Ala-$NH_2$, Gly, or Gly-$NH_2$; provided that at least one of $A^2$ or $A^3$ must be D-Phe; the method comprising the steps of providing $A^8$ protected with a protecting group, reacting said protected $A^8$ with resin to form resin-bound protected $A^8$, washing said resin-bound protected $A^8$, adding successively to said resin-bound protected $A^8$, protected $A^7$, protected $A^6$, protected $A^5$, protected $A^4$, protected Tyr, protected Ser, protected $A^3$, protected $A^2$ and protected $A^1$, acetylating the resulting product, and treating said product to remove said resin.

**Patentansprüche**

1. Decapeptid der Formel N-Ac-$A^1$-$A^2$-$A^3$-Ser-Tyr-$A^4$-$A^5$-$A^6$-$A^7$-$A^8$, worin $A^1$ D-β-Nal, D-p-X-Phe (worin X Cl, F, Br, oder $CH_3$ ist), D-Trp oder D-Phe ist; $A^2$ für D-p-X-Phe (worin X Cl, F, Br, oder $CH_3$ bedeutet), D-Phe oder D-Tyr steht; $A^3$ D-Trp, D-Phe oder D-Tyr ist; $A^4$ D-Arg, D-β-Naphthyl-Ala, D-Trp, D-Lys, D-homo-Arg, D-Diäthyl-homo-Arg oder D-p-X-Phe darstellt (worin X für Cl, F, Br oder $CH_3$ steht); $A^5$ Phe oder Tyr ist; $A^6$ Arg oder Lys bedeutet; $A^7$ Pro oder OH-Pro darstellt; und $A^8$ D-Ala, D-Ala-$NH_2$, Gly, oder Gly-$NH_2$ ist; mit der Maßgabe, daß wenigstens eines von $A^2$ oder $A^3$ D-Phe sein muß; oder ein pharmazeutisch verträgliches Salz desselben.

2. Decapeptid nach Anspruch 1, worin $A^1$ D-β-Nal ist, $A^2$ fur D-p-Cl-Phe steht, $A^3$ D-Phe bedeutet, $A^4$ D-Arg darstellt, $A^5$ Phe ist, $A^6$ für Arg steht, $A^7$ Pro bedeutet und $A^8$ D-Ala ist; oder ein pharmazeutisch verträgliches Salz desselben.

3. Decapeptid nach Anspruch 1, worin $A^1$ D-β-Nal bedeutet, $A^2$ für D-Phe steht, $A^3$ D-Phe ist, $A^4$ D-Arg darstellt, $A^5$ Phe ist, $A^6$ Arg bedeutet, $A^7$ für Pro steht, und $A^8$ D-Ala ist; oder ein pharmazeutisch verträgliches Salz desselben.

4. Therapeutische Zusammensetzung, die eine therapeutisch wirksame Menge eines Decapeptides nach einem der Ansprüche 1 bis 3 oder eines pharmazeutisch verträglichen Salzes desselben zusammen mit einer pharmazeutisch verträglichen Trägersubstanz enthält.

5. Zusammensetzung nach Anspruch 4, worin die genannte Zusammensetzung in Form einer Pille, Tablette, Kapsel, Flüssigkeit oder Tablette mit verzögerter Freisetzung für die perorale Verabreichung; in Form eines flüssigen Sprühmittels für die nasale Verabreichung; in Form einer Flüssigkeit, die dazu geeignet ist, intravenös, subkutan, parenteral oder intraperitoneal verabreicht zu werden; in Form eines biologisch erodierbaren, biologisch kompatiblen Implantates; oder in Form einer transdermalen Auflage hergestellt wird.

6. Zusammensetzung nach Anspruch 4, worin die genannte Zusammensetzung in Form einer injizierbaren Suspension hergestellt wird, die das genannte Decapeptid oder ein pharmazeutisch verträgliches Salz desselben und eine biologisch erodierbare, biologisch kompatible Polymermatrix enthält, die dazu geeignet ist, die verzögerte Freisetzung des genannten Decapeptides oder Salzes zu bewirken.

7. Decapeptid nach einem der Ansprüche 1 bis 3 oder sein pharmaceutisch verträgliches Salz oder eine Zusammensetzung nach einem der Ansprüche 4, 5 oder 6, für die Verwendung bei der Behandlung von verfrühter Pubertät, von hormonabhängigen Tumoren, z.B. malignen und benignen, Prostata-, Brust-, Eierstock- und Testikeltumoren, Hirsutismus, Akne, Amenorrhöe, Endometriose, Eierstock- und Brustzystenerkrankungen, zur Verhütung des Wachstums von Brusttumoren, zur Hemmung der LH-RHinduzierten Freisetzung von Geschlechtshormonen, zur Regelung der gonatropin-luteinisierenden Hormone den menschlichen Klimakteriums und der follikelstimulierenden Hormone während der Peri- und Postklimakteriumsperioden bei Frauen oder zur Empfängnisverhütung bei Frauen.

8. Verfahren zur Herstellung eines Peptides der Formel N-Ac-$A^1$-$A^2$-$A^3$-Ser-Tyr-$A^4$-$A^5$-$A^6$-$A^7$-$A^8$, worin $A^1$ D-β-Nal, D-p-X-Phe (worin X für Cl, F, Br, oder $CH_3$ steht), D-Trp oder D-Phe bedeutet; $A^2$ D-p-X-Phe, (worin X Cl, F, Br oder $CH_3$ darstellt), D-Phe oder D-Tyr ist; $A^3$ D-Trp, D-Phe oder D-Tyr ist; $A^4$ D-Arg, D-β-Naphthyl-Ala, D-Trp, D-Lys, D-homo-Arg, D-Diäthyl-homo-Arg, oder D-p-X-Phe (worin X für Cl, F, Br oder $CH_3$ steht) ist; $A^5$ Phe oder Tyr darstellt; $A^6$ Arg oder Lys bedeutet; $A^7$ Pro oder OH-Pro ist; und $A^8$ D-Ala, D-Ala-$NH_2$, Gly oder Gly-$NH_2$ ist; mit der Maßgabe, daß wenigstens $A^2$ oder $A^3$ D-Phe sein muß; welches Verfahren die Schritte des Schützens von $A^8$ mit einer Schutzgruppe, Umsetzen des genannten geschützten $A^8$ mit einem Harz, um ein harzgebundenes geschützes $A^8$ zu bilden, Waschen des genannten harzgebundenen geschützten $A^8$, aufeinanderfolgende Zugabe zum geschützten $A^8$ von geschütztem $A^7$, geschütztem $A^6$, geschütztem $A^5$, geschütztem $A^4$, geschütztem Tyr, geschütztem Ser, geschütztem $A^3$, geschütztem $A^2$ und geschütztem $A^1$, Acetylieren des erhaltenen Produktes und Behandeln des genannten Produktes umfaßt, um das genannte Harz zu entfernen.

**Revendications**

1. Un décapeptide de formule générale:

N-Ac-$A^1$-$A^2$-$A^3$-Ser-Tyr-$A^4$-$A^5$-$A^6$-$A^7$-$A^8$,

dans laquelle
—$A^1$ est D-β-Nal, D-p-X-Phe (X étant Cl, F, Br ou $CH_3$), D-Trp, ou D-Phe;
—$A^2$ est D-p-X-Phe (X étant Cl, F, Br, ou $CH_3$), D-Phe, ou D-Tyr;
—$A^3$ est D-Trp, D-Phe, ou D-Tyr;
—$A^4$ est D-Arg, D-β-Naphthyl-Ala, D-Trp, D-Lys, D-homo-Arg, Di-diethyl-homo-Arg, ou D-p-X-Phe (X étant Cl, F, Br, ou $CH_3$);
—$A^5$ est Phe ou Tyr;
—$A^6$ est Arg ou Lys;
—$A^7$ est Pro ou OH-Pro; et
—$A^8$ est D-Ala, D-Ala-$NH_2$, Gly, ou Gly-$NH_2$;
avec la condition qu'au moins un parmi $A^2$ ou $A^3$ soit D-Phe;
ou un de ses sels pharmaceutiquement acceptables.

2. Le décapeptide selon la revendication 1, dans laquelle $A^1$ est D-β-Nal, $A^2$ est D-p-Cl-Phe, $A^3$ est D-Phe, $A^4$ est D-Arg, $A^5$ est Phe, $A^6$ est Arg, $A^7$ est Pro, et $A^8$ est D-Ala, ou un de ses sels pharmaceutiquement acceptables.

3. Le décapeptide selon la revendication 1, dans laquelle $A^1$ est D-β-Nal, $A^2$ est D-Phe, $A^3$ est D-Phe, $A^4$ est D-Arg, $A^5$ est Phe, $A^6$ est Arg, $A^7$ est Pro et $A^8$ est D-Ala ou un de ses sels pharmaceutiquement acceptables.

4. Une composition thérapeutique comprenant une quantité thérapeutiquement efficace d'un décapeptide tel que défini dans l'une des revendications 1 à 3 ou d'un de leurs sels pharmaceutiquement acceptables, associé à un excipient pharmaceutiquement acceptable.

5. La composition selon la revendication 4, dans laquelle ladite composition se présente sous forme de pillule, comprimé, gélule, liquide, ou de comprimé à libération retardée pour une administration par voie orale; sous forme d'un pulvérisateur liquide pour une administration par voie nasale; sous forme d'un liquide pouvant être administré par voies intraveineuse, sous-cutanée, parentérale ou intrapéritonéale; sous forme d'un implant biocompatible et bioérodable; ou sous forme d'un emplâtre transdermal.

6. La composition selon la revendication 4, dans laquelle ladite composition se présente sous forme de suspension injectable comprenant ledit décapeptide ou l'un de ses sels pharmaceutiquement acceptables et une matrice polymère bioérodable et biocompatible pouvant entraîner une libération retardée dudit décapeptide ou sel.

7. Un décapeptide tel que revendiqué dans l'une des revendications 1 à 3 ou son sel pharmaceutiquement acceptable ou une composition selon l'une des revendications 4, 5 ou 6 pour une utilisation dans le traitement de la puberté précoce, des tumeurs hormono-dépendantes, par exemple, les tumeurs prostatiques malignes et bénignes, les tumeurs mammaires, testiculaires ou ovariennes, de l'hirsutisme, de l'acné, de l'aménorrhée, de l'endométriose, des maladies cystiques mammaires et ovariennes; pour une utilisation dans prévention de la croissance des tumeurs mammaires, dans l'inhibition de la libération des hormones sexuelles induites par le LH-RH, pour une utilisation dans la régulation de la gonadotropine lutéinisante dans la ménopause humaine et dans la stimulation de la folliculine durant les périodes périménopausales et postménopausales chez la femme, ou pour une utilisation dans les contraceptifs féminins.

8. Une méthode de synthèse d'un peptide de formule générale:

N-Ac-$A^1$-$A^2$-$A^3$-Ser-Tyr-$A^4$-$A^5$-$A^6$-$A^7$-$A^8$,

dans laquelle
—$A^1$ est D-β-Nal, D-p-X-Phe (X étant Cl, F, Br, ou $CH_3$), D-Trp, ou D-Phe;
—$A^2$ est D-p-X-Phe (où X est Cl, F, Br, ou $CH_3$), D-Phe, ou D-Tyr;
—$A^3$ est D-Trp-, D-Phe, ou D-Tyr;
—$A^4$ est D-Arg, D-β-Naphtyl-Ala, D-Trp, D-Lys, D-homo-Arg, D-diéthyl-homo-Arg, ou D-p-X-Phe (où X est Cl, F, Br, ou $CH_3$);
—$A^5$ est Phe ou Tyr;
—$A^6$ est Arg ou Lys;
—$A^7$ est Pro ou OH-Pro; et
—$A^8$ est D-Ala, D-Ala-$NH_2$, Gly, ou Gly-$NH_2$;
avec la condition qu'au moins l'un parmi $A^2$ ou $A^3$ soit D-Phe;
la méthode comprenant les étapes suivantes:
—mise en place d'un groupement protecteur sur l'acide aminé $A^8$,
—réaction de l'acide aminé $A^8$ protégé sur la résine pour fixer le $A^8$ protégé,
—lavage du $A^8$ protégé et fixé,

—additions successives audit $A^8$ protégé et fixé, du $A^7$ protégé, du $A^6$ protégé, du $A^5$ protégé, du $A^4$ protégé, de Tyr protégé, de Ser protégé, du $A^3$ protégé, du $A^2$ protégé et du $A^1$ protégé,

—acétylation du produit obtenu et

—traitement dudit produit pour éliminer la résine.